# EUROPÄISCHE PATENTSCHRIFT

(11) **EP 2 194 925 B1**
(45) Veröffentlichungstag und Bekanntmachung des Hinweises auf die Patenterteilung: **06.11.2013**
(21) Anmeldenummer: 08801319.8
(22) Anmeldetag: 10.09.2008
(51) Int. Cl.: A61F 2/24, A61B 17/04

(54) **HERZKLAPPENSTENT**
HEART VALVE STENT
TUTEUR DE VALVULE CARDIAQUE

(30) Priorität: 13.09.2007 DE 102007043830
(43) Veröffentlichungstag der Anmeldung: 16.06.2010
(73) Patentinhaber: Lutter, Georg, 24105 Kiel (DE); Lozonschi, Lucian, Madison, WI 53717 (US)
(72) Erfinder: Lutter, Georg, 24105 Kiel (DE); Lozonschi, Lucian, Madison, WI 53717 (US)
(74) Vertreter: Patentanwälte Westphal, Mussgnug & Partner
(86) Internationale Anmeldenummer: PCT/DE2008/001515
(87) Internationale Veröffentlichungsnummer: WO 2009/033469

(56) Entgegenhaltungen:
- US-A1- 2003 078 652
- US-A1- 2005 075 727
- US-A1- 2007 050 020

## Beschreibung

Die Erfindung betrifft einen Herzklappenstent mit einem zur Aufnahme eines Herzklappenimplantats eingerichteten Abschnitt und mit einer Mehrzahl von proximal angeordneten Verankerungselementen.

Derartige Herzklappenstents sind in einer Vielzahl von Ausbildungen zum Ersatz fehlgebildeter oder krankhaft veränderter Herzklappen bekannt. Dabei wird die chirurgische Implantation von Herzklappenprothesen regelmäßig am kardioplegischen Herzen durchgeführt. Die alte, in ihrer Funktion beeinträchtigte Herzklappe wird herausgeschnitten und die zu implantierende Herzklappenprothese eingenäht.

Bei Vorliegen von Erkrankungen der Mitralklappe hingegen wird versucht, die alte Klappe trotz aufgetretener Fehlfunktion weitestgehend zu erhalten, damit der gesamte dynamische Mitralklappenapparat nicht zerstört wird. Das liegt daran, dass z.B. die an der Mitralklappe ansetzenden Sehnenfäden (*chordae tendineae*) für die Ventrikelfunktion sehr wichtig sind und daher möglichst nicht von der alten Mitralklappe losgelöst werden sollten.

Ideal ist es daher, die Mitralklappe (wenn sie nicht rekonstruiert werden kann) weitestgehend zur Seite zu drängen, um der Klappenprothese Platz zu machen. Der Platz spielt hier eine nicht so gravierende Rolle wie in dem Aortenannulus, bei dem eine Einengung viel leichter zu Stande kommt (z.B. beim Verdrängen der alten Aortenklappe bei rein perkutaner Implantation).

Die Sehnenfäden (*chordae tendineae*) der Mitralklappe sollten dabei nach Möglichkeit in ihrer Struktur erhalten bleiben, um die Ventrikelgeometrie und damit eine bestmögliche Funktion der linken Kammer weitestgehend zu erhalten bzw. zu erreichen. Hierbei ist es von enormer Bedeutung, dass das vordere (anteriore) Mitralklappensegel nicht einfach in den freien Raum Richtung linken Ventrikel gedrückt wird, sondern an den Mitralklappenannulus angeheftet wird, damit ein Vorwärts-Drängen des vorderen Mitralklappensegels in den Ausflusstrakt des linken Ventrikels (Sam-Phänomen: Systolic Anterior Movement) verhindert wird. Dieses ist außerordentlich wichtig, da es sonst schnell zu einer Linksherz-Dekompensation (massives Fehlverhalten des linken Ventrikels) kommen kann.

Chirurgisch wird also die alte Mitralklappe an den alten Annulus angeheftet, so dass ein ungehinderter Blutfluss durch die Klappe und durch die beiden anliegenden Herzkammern gegeben ist. Nach dem Zurückdrängen (dem Anheften der Klappe an den Annulus) wird die Herzklappenprothese chirurgisch in diesen Annulus durch Einnähen implantiert.

Diese aufwendige Methode geschieht obligatorisch unter zur Hilfenahme der Herz-Lungen-Maschine und kommt für Hochrisikopatienten regelmäßig nicht in Frage, sodass nach minimal-invasiven oder perkutanen Methoden zur Implantation einer Herzklappe gesucht wird.

So ist aus der DE 195 46 692 C2 oder in EP 1 469 797 B1 eine selbstexpandierbare Herzklappenprothese zur Implantation im menschlichen Körper über ein Kathetersystem mit einer Herzklappe und mit einem mit der Herzklappe verbundenen zusammenfaltbaren und expandierbaren Stent bekannt. Eine solche selbstexpandierbare Herzklappenprothese kann mit Hilfe eines Kathetersystems durch eine Leistenarterie bis hin zum Implantationsort am Herzen geführt werden. Nach Erreichen des Implantationsortes kann dann ein solcher Stent, der in seiner Längsrichtung aus mehreren relativ zueinander abwinkelbaren selbstexpandierenden Segmenten zusammengesetzt ist, sukzessiv entfaltet werden. Nach der Entfaltung kann die Herzklappenprothese mit Unterstützung von Verankerungshaken zumindest im herznahen Bereich im jeweiligen Blutgefäss verankert werden.

Eine weitere Vorrichtung zur Befestigung und Verankerung von Herklappenprothesen ist in der DE 100 10 074 A1 beschrieben, die im Wesentlichen aus drahtförmigen miteinander verbundenen Elementen gebildet wird. Verschieden ausgebildete Bügel werden hierbei eingesetzt, um eine sichere Befestigung und Abstützung einer Herzklappe zu erreichen.

Bei diesen bekannten Lösungen besteht jedoch die Gefahr einer Fehlimplantation von Herklappen durch fehlerhafte Positionierung und der fehlerhafte angulare Ausrichtung der zu implantierenden Herzklappenprothese.

Eine bessere Positionierung und angulare Ausrichtung kann durch den aus der EP 1 469 797 B1 bekannten Stent erreicht werden, bei dem sogenannte Stützbügel ausgebildet sind, die in die Aortentaschen einführbar sind und so eine definierte Entfernung zur Aortenklappe herstellen. Darüber hinaus besteht auch die Möglichkeit eine fehlgeschlagene Implantation einer Herzklappenprothese abzubrechen und den Klappenstent wieder in das Kathetersystem bzw. die sogenannte die Kartusche hineinzuziehen. Hierbei wird dann der gesamte Stent zusammengefaltet und in die Kartusche zurückgeführt. Dabei ist es möglich den Stent erneut heraus gleiten zu lassen, sodass eine gute Positionierung für den Klappenstent nach mehreren Positionierungsversuchen erreicht werden kann ("sliding technique").

Darüberhinaus sind auch Herzklappenstents aus der US 2005/075727 A1, der US 2007/050020 A1 und der US 2003/078652 A1 bekannt, bei denen der Stent unter anderem auch mit Verankerungsfäden im Herzen ausgerichtet und fixiert wird.

Ein weitaus größeres Problem für die optimale Platzierung der neuen Herzklappe im Stent (bzw. Klappenstent) besteht jedoch darin, dass die alte native Klappe in den meisten Fällen der zuvor beschriebenen Implantationstechnik nicht entfernt werden soll.

Dies führt jedoch dazu, dass die neue Klappe, die in eine alte, deformierte Klappe hineingedrückt (teilweise hineingequetscht) wird, in ihrer ursprünglichen Stentform abgewandelt wird. Dies liegt daran, dass der Implantationsort für den Klappenstent durch die Anatomie, den Zustand und die Beschaffenheit der alten nativen Klappe bestimmt wird (z.B. bei Klappensklerose oder -verkalkung der nativen Klappe).

Daher bestimmt der alte Klappenannulus mit den dazugehörigen veränderten Klappen/Taschen inwieweit und wohin sich die neue Klappe entfalten und damit in ihrer Form entwickeln kann. Somit ist also nicht nur die Verankerung/Positionierung wichtig, um eine optimale Klappenftunktion, sowie Vorhof- und Ventrikelfunktion zu erhalten, sondern auch das Einpassen des Klappenstents in den Neo-Annulus (alter Klappenannulus bildet diesen mit alter Klappe aus) und damit das Zurückdrängen der alten Klappe.

Ausgehend von den bei den bekannten Herzklappenstents auftretenden Problemen, ist es daher Aufgabe der Erfindung einen Herzklappenstent, insbesondere einen Mitralklappenstent für die minimal-invasive Transplantation, zu schaffen, der eine möglichst natürliche Funktionsausübung des Herzens ermöglicht.

Erfindungsgemäß wird die Aufgabe durch den Herzklappenstent mit den Merkmalen von Anspruch 1 gelöst. Die Unteransprüche geben vorteilhafte Ausgestaltungen der Erfindung wieder.

Grundgedanke der Erfindung ist es, einen Herzklappenstent zu schaffen, der die anatomischen Voraussetzungen für eine natürliche Funktionsausübung, ähnlich einem gesunden Herz, schafft. Dabei ist mit dem erfindungsgemäßen Herzklappenstent in der selbstexpandierend faltbaren Ausgestaltung eine minimal-invasive Operation ermöglicht, die eine exakte Positionierung und sichere Fixierung des Klappenstents gewährleistet. Dabei wird, insbesondere bei Ausbildung des erfindungsgemäßen Herzklappenstents als Mitralklappenstent, eine Spannung zwischen Mitralklappe und Ventrikel ähnlich der natürlichen Sehnenfadenspannung (der *chordae tendineae*) erzeugt und gleichzeitig dafür gesorgt, dass die Klappenanteile der alten Mitralklappe (insbesondere das anteriore Mitralklappensegel) nicht mehr den Durchfluss des Blutes stören.

Es ist also vorgesehen, dass der Klappenstent nach der Erfindung kathetergestützt durch eines der Herzkammern oder der angrenzenden, großen Gefäße des Herzens eingeführt wird, sich dann in einer der Herzkammern entfalten kann, wobei seine Verankerungselemente im Gewebe fixiert werden. Abschließend wird der Stent an der dem Stent gegenüberliegenden, subvalvulären Herzkammerwandung unter Ausbildung einer Spannung zwischen der Herzkammerwandung und den proximal, supravalvulär verankerten Verankerungselementen mit Verankerungsfäden (im Folgenden auch *neo-chordae* genannt) fixiert.

Die Fixierung der Verankerungsfäden in der distalen Herzkammerwandung stellt also zu den proximalen Verankerungselementen ein Widerlager dar, das beispielsweise durch einen Knoten oder durch ein anderes als Widerlager wirkendes Element gebildet wird. Dieses Widerlager kann bevorzugt auch als Fädenlängenstellelement ausgebildet sein.

Vorteile des erfindungsgemäßen Herzklappenstents sind also genaue und einfache Fixierung des Herzklappenstents bei minimal-invasiver Operation und verbesserte Kontraktilität des Herzens gegenüber herkömmlichen Klappenstents.

Bevorzugt sind die im Verhältnis zur Längsachse des erfindungsgemäßen Klappenstents axial ausgerichteten Verankerungsfäden mit ihrem einen Ende am Annulus des Herzklappenimplantats befestigt, sodass bei Ausbildung der Spannung zwischen Stent und Herzkammerwandung eine direkte Wirkung auf die Positionierung und angulare Ausrichtung der Klappe erreicht werden kann. Die Verankerungsfäden können jedoch auch am distalen Klappenstentumfang mit dem Stent verbunden sein. Die Verbindung zwischen Verankerungsfäden und Stent muss jedoch so vorgenommen sein, dass eine im Wesentlichen im Verhältnis zur Längsachse des Stents in axialer Richtung verlaufende Spannung zwischen proximalen Verankerungselementen und distalem Widerlager ausgebildet werden kann.

Nach einer weiteren bevorzugten Ausgestaltung der Erfindung weisen die Verankerungsfäden (*neo-chordae*) Elemente zur Längenverstellung der Verankerungsfäden auf, mit den die Länge der Verankerungsfäden zum Einstellen einer bestimmten Spannung zwischen Herzklappenstent und Herzwandung eingestellt werden kann. Dabei kann beispielsweise ein Fadenlängenstellelement für je einen Faden oder beispielweise ein Fadenlängenstellelement für alle Fäden gemeinsam vorgesehen sein. Das Fadenlängenstellelement ist bevorzugt klein ausgebildet und kann beispielsweise derart eingerichtet sein, dass das Element durch Aufwickeln einer überschüssigen Fadenlänge den Faden auf die gewünschte Länge verkürzt.

Bevorzugt ist auch die Ausgestaltung von in axialer Richtung elastischen Verankerungsfäden, diese auf Kontraktionsbewegungen des Herzens reagieren können, ohne dass eine zu große Fadenlänge vorliegt, die die Herzfunktion negativ beeinflussen könnte. Dabei ist die Fadenlänge so zu wählen, dass die Elastizität nicht vollständig auf Kosten der Spannung zwischen Verankerungselementen und Herzwandung geht.

Ist das Widerlager als Fädenlängenstellelement ausgebildet, ergibt sich eine besonders vorteilhafte Ausgestaltung, da hiermit auch eine Nacheinstellung der Spannung zwischen Verankerungselementen und Widerlager, also ein Nachspannen der Verankerungsfäden möglich ist, ohne dass das Herz geöffnet werden müsste.

Besonders bevorzugt ist die Struktur des Mitralklappenstents in der Ebene des Mitralklappenannulus im Wesentlichen oval oder U-förmig ausgebildet, dass kein Druck auf den LVOT (Left Ventricular Outflow Tract; linksventrikulärer Ausflusstrakt) und/oder Aortenannulus ausgeübt wird und damit eine Beeinträchtigung der Herzfunktion unterbleibt (Ma L, Tozzi P, Huber CH, Taub S, Gerelle G, von Segesser LK. Double-crowned valved stents for off-pump mitral valve replacement. Eur J Cardiothorac Surg. 2005 Aug; 28(2): 194-8; discussion 198-9.). Auch bleibt der subvalvuläre Apparat in seiner natürlichen Anatomie dadurch vollkommen erhalten und wird nicht beeinträchtigt (Boudjemline Y, Agnoletti G, Bonnet D, Behr L, Borenstein N, Sidi D, Bonhoeffer P. Steps toward the percutaneous replacement of atrioventricular valves an experimental study. J Am Coll Cardiol. 2005 Jul 19;46(2):360-5).

Dieser Klappenstent erhält eine dem natürlichen Mitralklappen-Apparat vollkommen angepasste, äußerst anschmiegende Form, so dass sich dieser konisch-zulaufende (kranial-caudale Achse) nicht ganz zirkuläre (oval-ähnlich in der transversalen Achse) Klappenstent sehr stark an die natürliche Form der Mitralklappe anlegen und anlehnen kann. Im Bereich des anterioren Mitralklappenannulus ist der Klappenstent flach und führt zu fast keinem Druck und zu keiner Einengung auf den LVOT. Im Bereich des posterioren Mitralklappenannulus ist er oval und bildet die Form des posterioren Annulus nach. Dieser Klappenstent bildet eine dünne, in der Länge (cranial-caudal) begrenzte Struktur, die sich in seiner Form vollkommen an die Mitralklappe anschmiegt und damit aussieht wie ein Negativabdruck im Bereich des natürlichen Mitralklappenannulus. Der Klappenstent berührt zwar die alte Mitralklappe und deren Annulus, jedoch belässt er diese in ihrer Anatomie fast vollkommen unverändert.

Die Erfindung wird im Folgenden anhand von in den beigefügten Zeichnungen abgebildeten besonders bevorzugten Ausführungsbeispielen näher erläutert. Es zeigen:
- Fig. 1: ein bevorzugtes Ausführungsbeispiel des erfindungsgemäßen Klappenstents in einer schematischen Seitenansicht;
- Fig. 2: das in Fig. 1 gezeigte Ausführungsbeispiel in Draufsicht von oben;
- Fig. 3: eine Draufsicht auf mehrere besonders bevorzugt ausgebildete Klappenstents nach der Erfindung;
- Fig. 4: eine Draufsicht auf ein Ausführungsbeispiel von unten;
- Fig. 5: eine schematische Ansicht zur Verdeutlichung der minimal-invasiven Transplantation eines Mitralklappenstents nach der Erfindung in einer ersten Phase bei Einbringen des erfindungsgemäßen Mitralklappenstents an den Transplantationsort;
- Fig. 6: eine schematische Ansicht zur Verdeutlichung der minimal-invasiven Transplantation eines Mitralklappenstents nach der Erfindung in einer zweiten Phase nach Positionierung der Mitralklappe;
- Fig. 7: eine schematische Ansicht zur Verdeutlichung der minimal-invasiven Transplantation eines Mitralklappenstents nach der Erfindung nach Abschluss der Fixierung der Verankerungsfäden außerhalb des Apex der linkventrikulären Herzwandung;
- Fig. 8: eine schematische Ansicht einer alternativen, intracardialen Fixierung der Verankerungsfäden im Bereich der Papillarmuskeln;
- Fig. 9: eine schematische Ansicht eines im Aortenannulus fixierten Herzklappenstents;
- Fig. 10: eine schematische Ansicht eines in der Pulmonalposition fixierten Herzklappenstents;
- Fig. 11: eine schematische Ansicht eines in der Trikuspidalposition fixierten Herzklappenstents;
- Fig. 12: einen Klappenstent in einer schematischen Seitenansicht ohne Herzklappe und Verankerungsfäden; und
- Fig. 13: eine schematische, dorsale, intracardiale Ansicht eines in der Mitralposition fixierten Herzklappenstents.

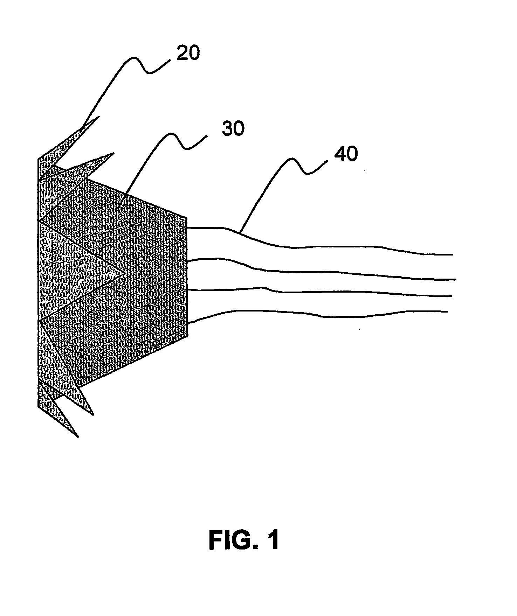

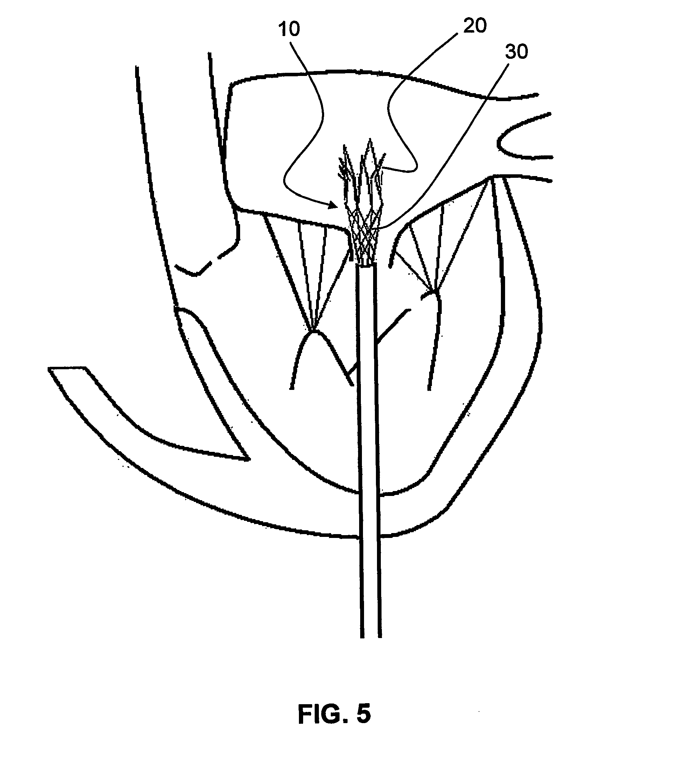

Die Fig. 1 bis 8 zeigen den erfindungsgemäßen Stent zur Implantation und Befestigung von Herzklappenprothesen in verschiedenen Ansichten zur Verdeutlichung des Aufbaus des Stents und der räumlichen Verhältnisse der einzelnen Stentabschnitte zueinander im entfalteten (Fig. 1-4 und 6-8) und im gefalteten Zustand (Fig. 5).

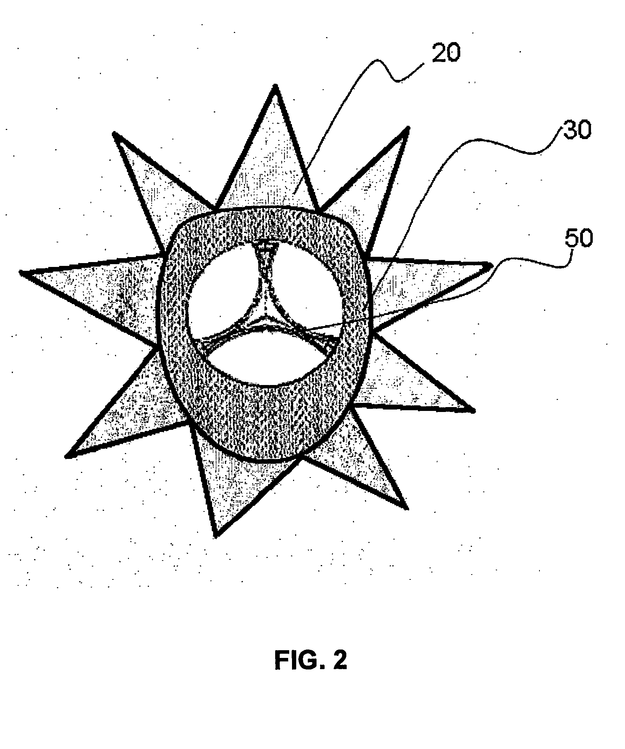

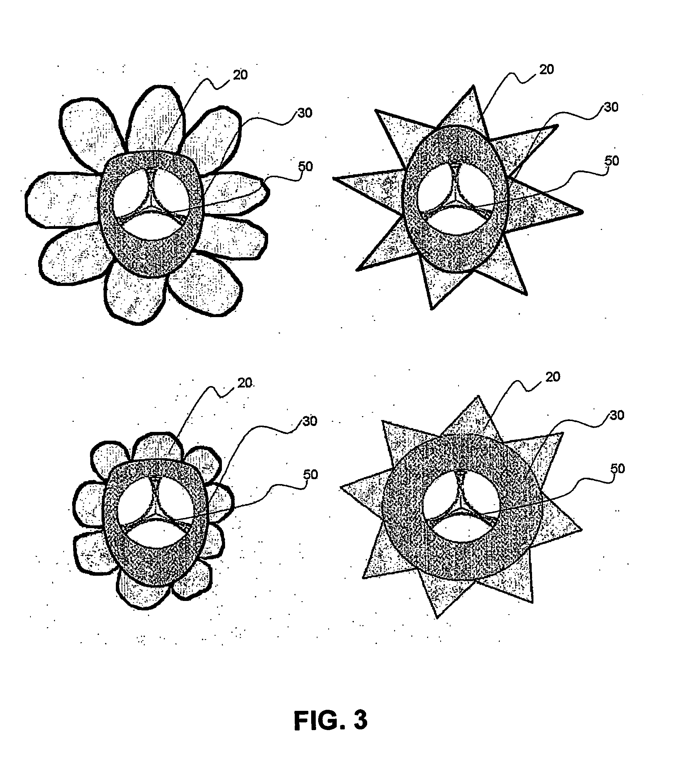

Fig. 1 zeigt einen faltbaren Mitralklappenstent 10 nach der Erfindung in einer perspektivischen Lateralansicht. Der Stent 10 weist im Wesentlichen drei Abschnitte auf: Proximal (supravalvulär) sind am Stent 10 kreisförmig eine Vielzahl von gezackt- oder bogenförmigen (Fig. 3) Verankerungselementen 20 angeordnet, die den Klappenstent 10 im implantierten Zustand supravalvulär (bzw. atrial) verankern. Distal benachbart angeordnet befindet sich der konisch- und im Querschnitt ovalartig geformte, zum LVOT bevorzugt abgeflachte Stentkörper 30 (vgl. Fig. 2).

Der Stentkörper 30 bildet eine korb- oder trapezähnliche Figur, die sich an den Mitralklappenannulus anschmiegt und sich in Richtung des linken Ventrikels erstreckt. Gehalten wird dieser Stent 10 im Atrium durch seine konisch zulaufende Form und durch die atrialen Verankerungselemente 20. In diesen Stentkörper 30 kann eine zwei- oder dreizipflige Klappe 50 integriert sein.

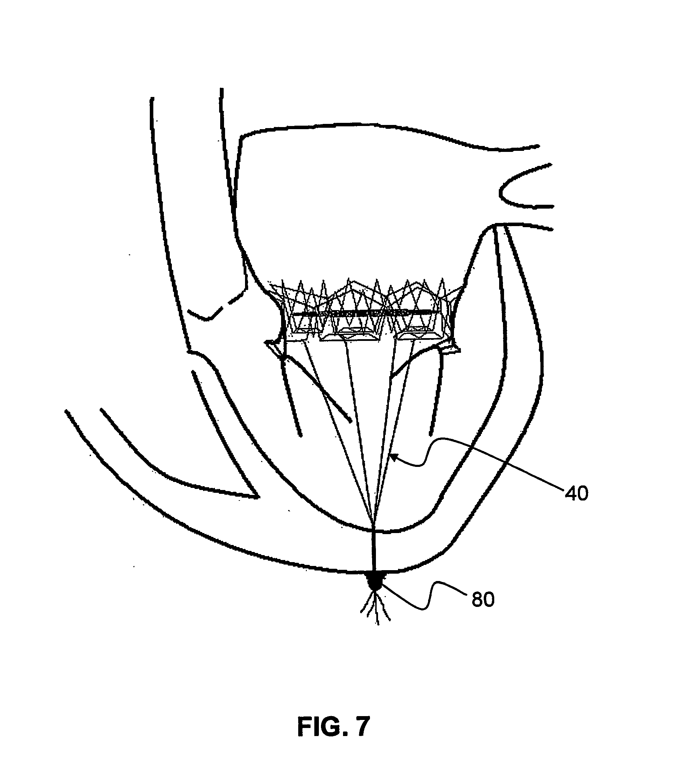

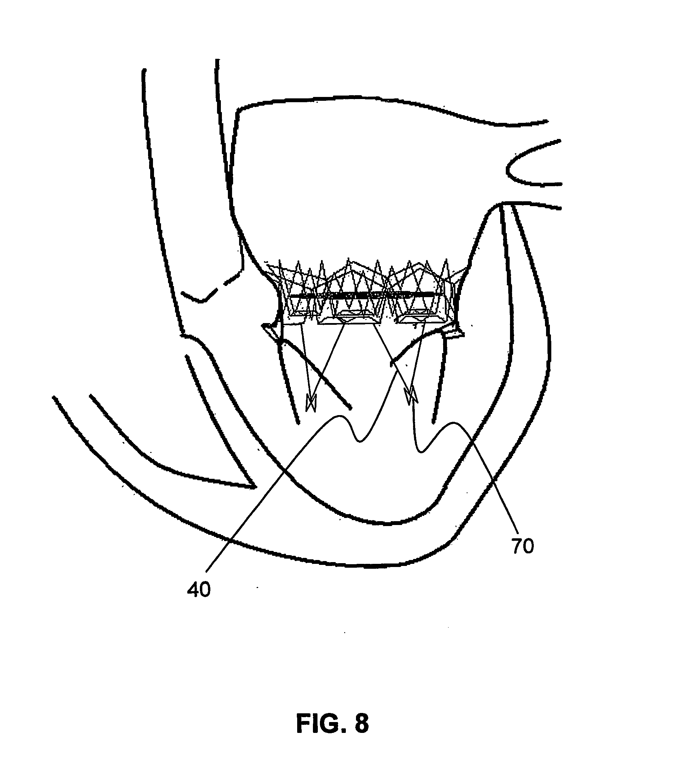

An dem Stentkörper 30 befinden sich distal (zum linken Ventrikel hin) Verankerungsfäden 40, die distal von diesem Stentkörper 30 zur Verankerung des gesamten Stents 10 eingerichtet sind. Diese Verankerungsfäden 40 sorgen für eine Verankerung in der gegenüberliegenden Ventrikelwand oder z.B. im Bereich des Papillarmuskels (proximale, medialer oder distaler Papillarmuskelanteil); vgl. Fig. 7 und 8. Diese Verankerungsfäden 40 können mittels eines adjustierbaren Fadenlängenstellelements 70 positioniert und mit der optimalen Länge eingestellt werden, so dass der Herzklappenstent 10 dann fixiert und verankert werden können.

Fig. 2 zeigt den Stent 10 in einer Draufsicht. Dabei ist zu erkennen, dass der Stent 10 einen Neo-Annulus, bzw. Stentkörper 30 ausbildet, in den die Herzklappenprothese 50 eingesetzt und an dem sie befestigt werden kann. Weiterhin ist zu erkennen, dass der erfindungsgemäße Stent 10 in Bezug auf die Vielzahl von supravalvulären (atrialen) Stentbügel 20 asymmetrisch ausgebildet sein kann. Dieses ist daran zu erkennen, dass der Stentkörper 30 in dieser Abbildung ovalartig ausgebildet und an einer Seite abgeflacht, also im Wesentlichen U-förmig ausgebildet ist, so dass er an dieser abgeflachten Stelle in die Richtung des LVOTs eingesetzt werden kann. Diese Abflachung bewirkt, dass an dieser Stelle kein Druck auf den LVOT und die Aortenklappe durch den selsbstexpandierbaren Stent ausgeübt werden kann, wenn der Stent 10 z.B. in die Mitralposition eingesetzt wird. Weitere bevorzugte Ausgestaltungen des Stents 10 nach der Erfindung sind in Fig. 3 gezeigt.

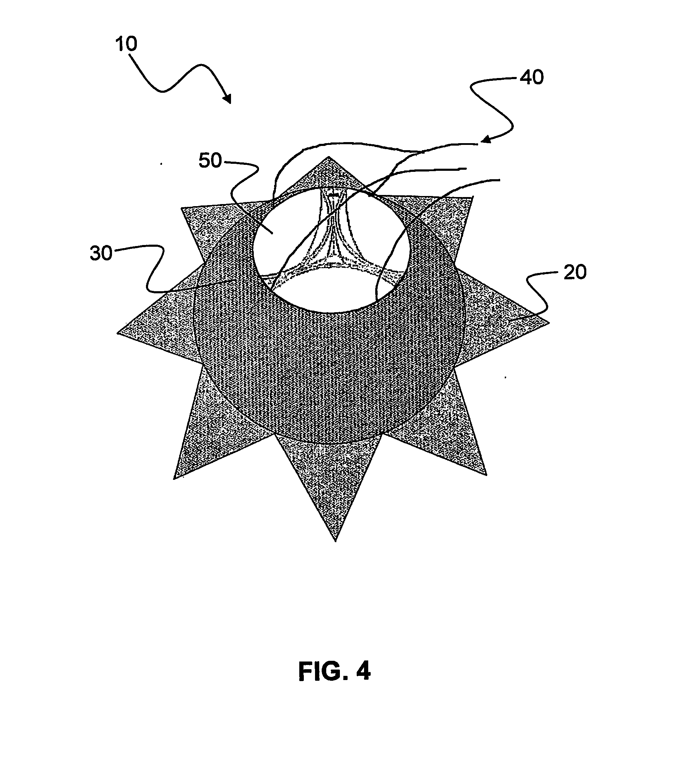

Fig. 4 zeigt den erfindungsgemäßen Stent 10 in einer Unteransicht. Hieraus wird deutlich, dass sich der Durchmesser von dem atrialen Anteil zum ventrikulären Anteil des Stentkörpers 30 verringert, so dass dieser von lateral aussieht wie ein Kegelstumpf (vgl. Fig. 1). Sowohl die Befestigungselemente 20 als auch der Stentkörper 30 können mit Stoff (z.B. Kunststoff, Perikard, PTFE oder Goretex, etc.) bezogen sein, um eine verbesserte Abdichtung zwischen der Herzklappenprothese 50, Stentkörper 30 und der umgebenden Herzstruktur zu erreichen. Diese Abdichungsmembran wird dann zwischen Herzklappenprothese 50 und Stentkörper 30 oder auf die atrialen Stentstreben 20 ein-, bzw. aufgezogen, um eine optimale Abdichtung der Klappe zwischen den beiden Herzkammern zu erreichen.

In den Fig. 5 bis 7 und 8 wird die retrograde transapikale Implantation eines Klappenstents beschrieben. Der retrograde transaortale als auch der antegrade transatriale Zugangsweg kann alternativ durchgeführt werden. Die Platzierung des Klappenstents mit einem gefalteten Klappenstent über dem alten Mitralklappen-Annulus ist in Fig. 5 gezeigt. Nach erfolgreicher Orientierung mittels Markierungen am Klappenstent 10 (nicht gezeigt) kann langsam mit der Entfaltung (z.B. bevorzugt selbstexpandierend) der atrialen Verankerungselemente 20 begonnen werden. Die Positionierung im linken Atrium sollte so geschehen, dass die abgeflachte Seite des Stentkörpers 30 in die Richtung es LVOT (Aortenklappe) zu liegen kommt. Der Stent wird dann weiter expandiert.

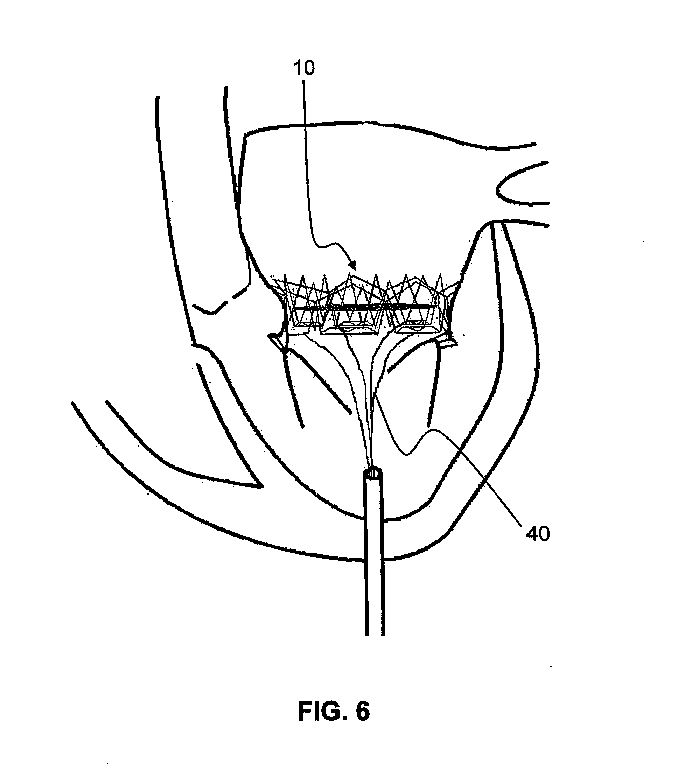

Fig. 6 zeigt den expandierten Klappenstent 10 in dem links-atrioventrikularen Einflusstrakt. Verankerungsfäden 40 werden an, in oder außerhalb der Herzwand adjustiert und später - wie in Fig. 7 dargestellt - mit Hilfe des bevorzugt als Fädenlängenstellelement ausgebildeten Widerlagers 80 fixiert. Während des Adjustierens der Länge der Verankerungsfäden 40 erfolgt u.a. auch eine Visualisierung des Mitralklappenapparates (z.B. Echo, CT oder NMR), sodass der Annulus mittels des neuen Stents 10 optimal in Richtung Ventrikelwand gezogen werden kann, kein paravalvuläres Leck mehr besteht, der Stent 10 gut fixiert ist und der Mitralklappen-annulus und -apparat vorteilhaft die linksventrikuläre Funktion unterstützt.

Alternativ zu Fig. 7 können die Verankerungsfäden 40 auch an den Papillarmuskeln befestigt werden (siehe Fig. 8), so dass diese Fäden 40 *neo-chordae* darstellen und die Funktion der außer Funktion gesetzten *chordae tendineae* übernehmen können. Die Befestigung der Verankerungsfäden 40 an der Herzwandung erfolgt jeweils mittels eines Widerlagers 80, das als Knoten oder auch als eigenständiges Element ausgebildet sein kann. Auch ist es möglich, dass die ventrikulären Verankerungsfäden 40 nicht nur am Stentkörper 30, sondern auch an der integrierten Klappe selbst angebracht werden. Auch können diese kaudalen Verankerungsfäden 40 an jedem anderen Punkt im Ventrikel fixiert werden.

Die Fig. 7 zeigt die vollendete Positionierung und Fixierung des Stents 10. Nach Längen und Lagebestimmung der einzelnen Verankerungsfäden 40 werden diese mittels des adjustierbaren Fadenlängenstellelements 70 z.B. in der linksventrikulären Wand fixiert. Das Fadenlängenstellelement 70 dient der optimalen Einstellung der Länge und der Position des Klappenstents 10 und damit der Klappenprothese 50. Unterschiedliche Fäden 40 können unterschiedliche Längen und Befestigungspositionen im Ventrikel aufweisen.

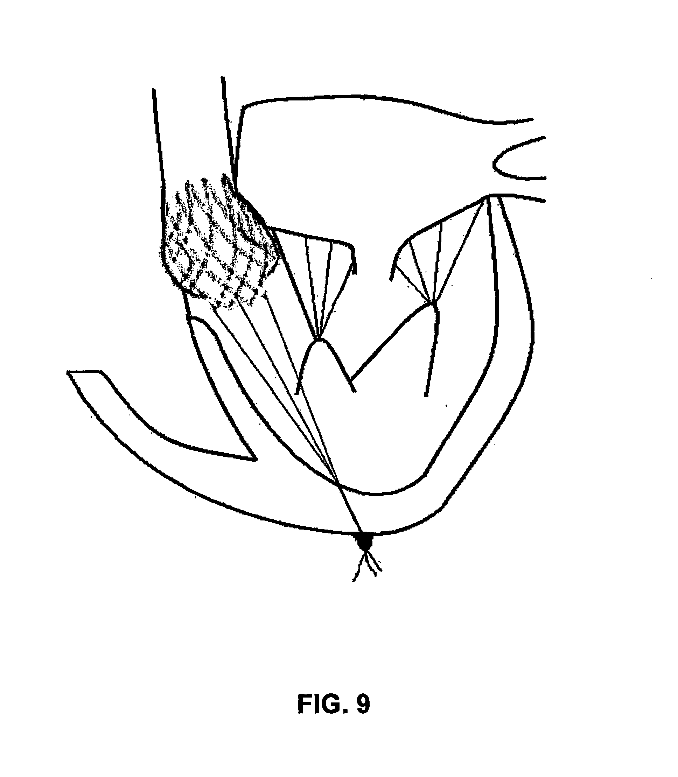

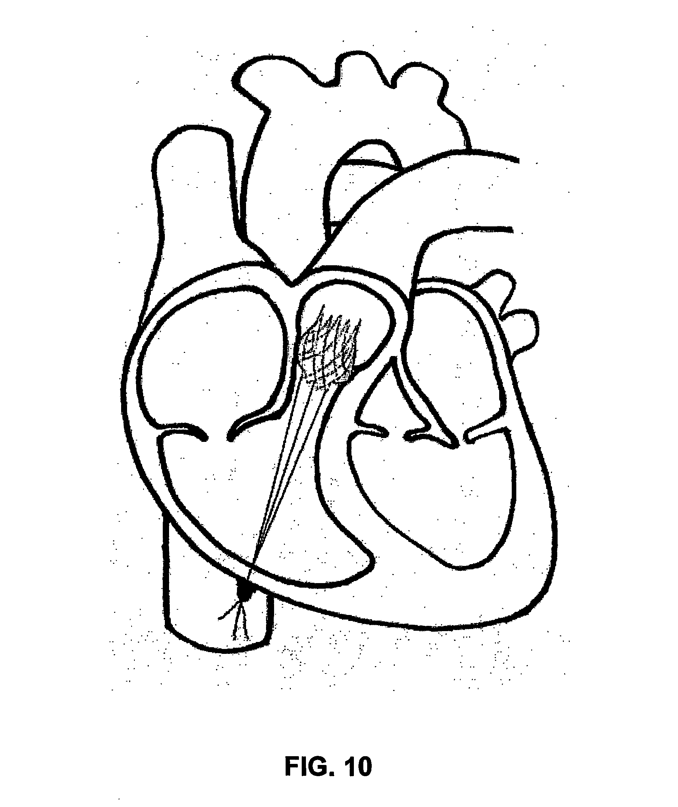

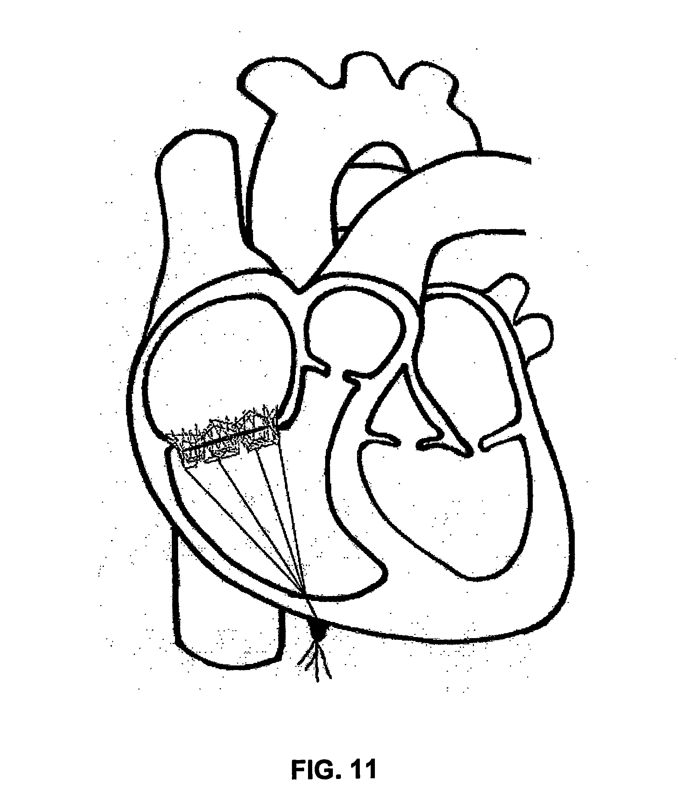

Die Fig. 9 bis 11 zeigen weitere Beispiele für den Einsatz des Herzklappenstents 10 wobei der Stent 10 an die jeweilige Anatomie angepasst ist (für die Aorten- und Pulmonalklappenposition eine eher zirkuläre Form (vgl. Fig. 3) und für die Tricuspidalposition eine eher ovale Form).

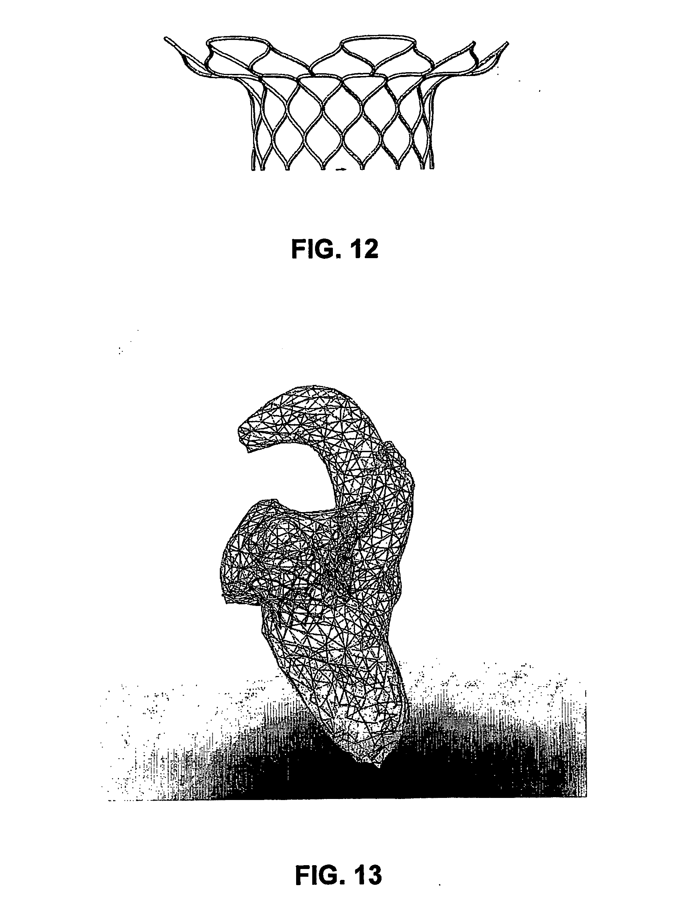

Fig. 12 zeigt einen Klappenstent in einer schematischen Seitenansicht, der zur besseren Übersichtlichkeit ohne Herzklappe und Verankerungsfäden dargestellt ist.

Zur Verdeutlichung der Lage des Klappenstents aus Fig. 12 in situ zeigt Fig. 13 eine schematische, dorsale, intracardiale Ansicht eines in der Mitralposition fixierten Herzklappenstents. Beachte das gute Anschmiegen des Klappenstents an die linksatriale Umgebung. Abstände zwischen der linksatrialen Wand / Mitralannulus und dem Klappenstent werden so vermieden. Herzklappe und Verankerungsfäden zur Ventrikelspitze sind zur Vereinfachung der Darstellung weggelassen worden.

## Patentansprüche

1. Herzklappenstent (10) mit einem zur Aufnahme eines Mitralklappenimplantats (30) eingerichteten Abschnitt und mit einer Mehrzahl von proximal angeordneten Verankerungselementen (20),
und einer Mehrzahl von Verankerungsfäden (40), die mit ihrem einen Ende am Stent (10) befestigt sind, und
einem die Verankerungsfäden (40) mit ihrem anderen Ende an der distalen Herzkammerwandung unter Ausbildung einer Spannung zwischen der Herzkammerwandung und den proximal verankerten Verankerungselementen (20) befestigenden Widerlager (80),
**dadurch gekennzeichnet, dass**
der Mitralklappenstent (10) als Kegelstumpf ausgebildet ist.

2. Herzklappenstent (10) nach Anspruch 1, **dadurch gekennzeichnet, dass** die Verankerungsfäden (40) am Annulus des Mitralklappenimplantats (30) befestigt sind.

3. Herzklappenstent (10) nach einem der vorhergehenden Ansprüche, **gekennzeichnet durch** wenigstens ein zur Längeneinstellung wenigstens eines Verankerungsfadens (40) eingerichtetes Fadenlängenstellelement (70).

4. Herzklappenstent (10) nach einem der vorhergehenden Ansprüche, **dadurch gekennzeichnet, dass** das Widerlager (80) als Fadenlängenstellelement ausgebildet ist.

5. Herzklappenstent (10) nach Anspruch 4, **dadurch gekennzeichnet, dass** der Herzklappenstent (10) in der Ebene des Mitralklappenannulus im Wesentlichen oval oder U-förmig ausgebildet ist.

6. Herzklappenstent (10) nach einem der vorhergehenden Ansprüche, **dadurch gekennzeichnet, dass** zwischen dem Herzklappenimplantat (30) und dem Stent (10) eine Abdichtungsmembran angeordnet ist.

7. Herzklappenstent (10) nach einem der vorhergehenden Ansprüche, **dadurch gekennzeichnet, dass** zwischen den Verankerungselementen (20) und dem Herzklappenimplantat (30) eine Abdichtungsmembran angeordnet ist.

8. Herzklappenstent (10) nach einem der vorhergehenden Ansprüche, **dadurch gekennzeichnet, dass** wenigstens ein Verankerungselement (20) aus einer Formgedächtnislegierung besteht.

## Claims

1. A hart valve stent (10) having a section equipped to receive a mitral valve implant (30) and a plurality of proximally disposed anchoring elements (20), and a plurality of anchoring threads (40), which one end thereof is attached to the sent (10), and
a thrust bearing (80) securing the anchoring threads (40) with the other end thereof to the distal heart chamber wall to provide tension between the heart chamber wall an the proximally anchored anchoring elements (20),
**characterized in that** the mitral valve stent (10) is formed as a truncated cone.

2. Heart valve stent (10) according to claim 1, **characterized in that** the anchoring threads (40) are affixed to the annulus of the mitral valve implant (30).

3. Heart valve stent (10) according to one of the previous claims, **characterized by** at least one suture-length regulatory element (70) for adjusting the length of at least one anchoring suture (40).

4. Heart valve stent (10) according to one of the previous claims, **characterized in that** thrust bearing (80) is designed as a suture-length regulatory element.

5. Heart valve stent (10) according to claim 4, **characterized in that** in the plane of the mitral valve the heart valve stent (10) annulus substantially is oval or ushaped.

6. Heart valve stent (10) according to one of the previous claims, **characterized by** a sealing membrane arranged between the heart valve implant (30) and the stent (10).

7. Heart valve stent (10) according to one of the previous claims, **characterized by** a sealing membrane arranged between the anchoring elements (20) and the heart valve implant (30).

8. Heart valve stent (10) according to one of the previous claims, **characterized in that** at least one anchoring element (20) is made from a shape memory alloy.

## Revendications

1. Stent pour valvule cardiaque (10) comportant un tronçon conçu pour la réception d'un implant de valvule mitrale (30) ainsi qu'un ensemble d'éléments d'ancrage (20) montés à son extrémité proximale et un ensemble de fils d'ancrage (40) qui sont fixés au stent (10) à l'une de leurs extrémités et, une butée (80) permettant de fixer les fils d'ancrage (40) par leur autre extrémité à la paroi distale du ventricule en créant une tension entre la paroi du ventricule et les éléments d'ancrage (20) à ancrage proximal,
**caractérisé en ce que**
le stent de valvule mitrale (10) est réalisé sous la forme d'un tronc de cône.

2. Stent pour valvule cardiaque (10) conforme à la revendication 1,
**caractérisé en ce que**
les fils d'ancrage (40) sont fixés à l'annulus de l'implant de valvule mitrale (30).

3. Stent pour valvule cardiaque (10) conforme à l'une des revendications précédentes,
**caractérisé par**
au moins un élément de réglage de la longueur des fils (70) réalisé pour permettre le réglage de la longueur d'au moins l'un des fils d'ancrage (40).

4. Stent pour valvule cardiaque (10) conforme à l'une des revendications précédentes,
**caractérisé en ce que**
la butée (80) est réalisée sous la forme d'un élément de réglage de la longueur des fils.

5. Stent pour valvule cardiaque (10) conforme à la revendication 4,
**caractérisé en ce que**
le stent pour valvule cardiaque (10) est réalisé essentiellement en forme ovale ou en forme de U dans le plan de l'annulus de la valvule mitrale.

6. Stent pour valvule cardiaque (10) conforme à l'une des revendications précédentes,
**caractérisé en ce qu'**
une membrane d'étanchéité est montée entre l'implant pour valvule cardiaque (30) et le stent (10).

7. Stent pour valvule cardiaque (10) conforme à l'une des revendications précédentes,
**caractérisé en ce qu'**
une membrane d'étanchéité est montée entre les éléments d'ancrage (20) et l'implant pour valvule cardiaque (30).

8. Stent pour valvule cardiaque (10) conforme à l'une des revendications précédentes,
**caractérisé en ce qu'**
au moins un élément d'ancrage (20) est réalisé en un alliage à mémoire de forme.
